# EUROPÄISCHE PATENTSCHRIFT

(11) **EP 3 965 850 B1**
(45) Veröffentlichungstag und Bekanntmachung des Hinweises auf die Patenterteilung: **17.09.2025**
(21) Anmeldenummer: 20725657.9
(22) Anmeldetag: 05.05.2020
(51) Int. Cl.: A61M 5/178, A61M 5/315

(54) **DOSIER- UND AUSSCHÜTTMECHANISMUS FÜR EINE INJEKTIONSVORRICHTUNG**
DOSING AND DISPENSING MECHANISM FOR AN INJECTION DEVICE
MÉCANISME DE DOSAGE ET DE DÉVERSEMENT POUR UN DISPOSITIF D'INJECTION

(30) Priorität: 07.05.2019 EP 19172976
(43) Veröffentlichungstag der Anmeldung: 16.03.2022
(73) Patentinhaber: Ypsomed AG, 3401 Burgdorf (CH)
(72) Erfinder: HOSTETTLER, Patrick, 3415 Hasle (CH); SCHEURER, Simon, 3006 Bern (CH); HIRSCHEL, Jürg, 3007 Bern (CH)
(74) Vertreter: Meier Obertüfer, Jürg
(86) Internationale Anmeldenummer: PCT/EP2020/062388
(87) Internationale Veröffentlichungsnummer: WO 2020/225239

(56) Entgegenhaltungen:
- US-A1- 2016 045 664
- US-A1- 2018 008 783
- US-B2- 9 005 171

## Beschreibung

### TECHNISCHES GEBIET

Die vorliegende Erfindung betrifft das Gebiet der medizinischen Injektionsvorrichtungen zur Verabreichung von flüssigen Substanzen insbesondere von Medikamenten oder medizinischen Substanzen wie Insulin- und Hormonpräparationen. Die Erfindung bezieht sich auf eine Injektionsvorrichtung mit einem Dosier- und Ausschüttmechanismus.

### HINTERGRUND DER ERFINDUNG

Bekannte Injektionsgeräte umfassen üblicherweise eine Antriebshülse zum Antreiben einer Kolbenstange um die flüssigen Substanz aus einer Karpule oder einem Produktbehälter auszuschütten, ein Betätigungselement oder Dosierelement zum Einstellen einer zu verabreichenden Dosis und ein Element zum Anzeigen der eingestellten Dosis.

Um eine Dosis einzustellen, dreht oder zieht der Benutzer am Betätigungs- oder Dosierelement, welches sich dabei aus dem Gehäuse des Injektionsgeräts herausschraubt bzw. herausbewegt. Damit die eingestellte Dosis ausgeschüttet werden kann, drückt der Benutzer an einem proximalen Ende des Injektionsgeräts direkt auf das Betätigungselement oder auf einen Ausschüttknopf und erzeugt eine Kraft in distaler Richtung, wodurch das Betätigungselement in das Gehäuse eingeschraubt oder eingeschoben wird. Im Unterschied zum Einstellen einer Dosis überträgt beim Ausschütten das Betätigungselement die Dreh- oder Schiebebewegung auf die Antriebshülse, welche dadurch die Kolbenstange antreibt und die Substanz aus der Karpule ausschüttet. Je nach Ausführung ist dabei die Kolbenstange drehbar oder nur verschiebbar relativ zum Gehäuse gelagert.

Um eine versehentlich zu hoch eingestellte Dosis zu korrigieren, kann der Benutzer das Betätigungselement in das Gehäuse zurückdrehen oder zurückschieben. Bei dieser Korrekturbewegung wie auch bei der Handhabung des Injektionsgeräts zwischen den Verabreichungen muss sichergestellt werden, dass sich die Position der Kolbenstange nicht ungewollt relativ zur Karpule verstellen kann, insbesondere dass sich die Kolbenstange nicht von der Karpule wegbewegt.

Eine Möglichkeit ein ungewolltes Verstellen zu verhindern, ist die Verwendung einer sogenannten Rückdrehsicherung. Diese verhindert entweder direkt das Rotieren der Kolbenstange entgegen der Rotationsrichtung zum Ausschütten der Substanz oder die Rückdrehsicherung greift am antreibenden Antriebselement an, so dass eine Rückbewegung der Kolbenstange indirekt verhindert wird.

Alternativ dazu kann ein ungewolltes Verstellen der Kolbenstange mittels Selbsthemmung im Gewinde verhindert werden, insbesondere wenn die Kolbenstange mehrere Gewinde mit unterschiedlichen Steigungen aufweist.

So kann beispielsweise die Kolbenstange mit einem Linksgewinde mit der Antriebshülse in Gewindeeingriff stehen und gleichzeitig ein Rechtsgewinde für den Eingriff mit dem Gehäuse umfassen. Wird in diesem Fall beim Einstellen einer Dosis die Antriebshülse im Linksgewinde herausgeschraubt, wird die Kolbenstange nicht relativ zum Gehäuse bewegt, da diese mit dem rechtssteigenden Gewinde in Eingriff steht. Diese Sicherung der Kolbenstange mit unterschiedlichen Gewinden hat den Vorteil, dass keine speziellen Elemente wie beispielsweise eine Ratsche für das Verhindern einer ungewollten Drehung der Kolbenstange vonnöten sind. Zudem ist beim Ausschütten kein zusätzlicher Kraftaufwand erforderlich, um einen Reibungswiderstand Sicherungselements wie einer Ratsche zu überwinden.

Die WO 2014/033195 offenbart einen Mehrweg-Injektionspen mit einer Kolbenstange, welche ein erstes und ein zweites, gegenläufiges Gewinde umfasst. Mit dem ersten Gewinde ist die Kolbenstange im Gehäuse eingeschraubt. Mit dem zweiten Gewinde steht eine Antriebshülse in Gewindeeingriff. Der Injektionspen umfasst zudem ein Klickmechanismus mit einem distalen und einem proximalen Klickring, welche je axiale Zähne aufweisen. Beim Einstellen einer Dosis werden die Zähne relativ zueinander bewegt, so dass ein Klickgeräusch entsteht. Zudem sind beim Einstellen mittels einer Kupplung ein Ausschüttknopf, die Antriebshülse und eine Dosisanzeigehülse rotationsfest miteinander verbunden und werden aus dem Gehäuse herausgeschraubt. Die Kolbenstange bleibt dabei unbeweglich relativ zum Gehäuse und die Antriebshülse wird auf der Kolbenstange herausgeschraubt. Zum Ausschütten der Dosis wird der Ausschüttknopf gedrückt. Durch die erzeugte Axialkraft wird der rotationsfest mit dem Gehäuse verbundene proximale Klickring rotativ mit dem distalen Klickring gekoppelt, wodurch die Antriebshülse nur axial verschoben werden kann, während die Dosisanzeigehülse frei drehen kann und zurück ins Gehäuse geschraubt wird. Durch die axiale Verschiebung der Antriebshülse wird die Kolbenstange rotiert und das Produkt ausgeschüttet.

Dieser Mechanismus ist komplex und umfasst viele Einzelteile, was die Herstellung verteuert und ein aufwändiges Montieren bedingt. Zudem ist der Mechanismus für Mehrweg-Injektionspens ausgelegt, bei denen die Kolbenstange nach einer Verabreichung wieder in das Gehäuse zurückschiebbar sein muss.

US 9,005,171 B2 offenbart ein Injektionspen mit einem Rückstellmechanismus mit einer Antriebshülse, welche mittels Finger mit einer Kupplungshülse rotativ gekoppelt werden kann. Die Finger sind radial nach innen auslenkbar. Beim Dosiseinstellen werden Antriebshülse, Kupplungshülse und Dosiseinstellhülse rotiert. Beim Ausschütten werden durch eine axiale Bewegung der Kupplungshülse die Kupplungshülse und die Antriebshülse über die Finger zum Gehäuse gekoppelt und werden somit nur axial verschoben ohne zu rotieren.

US 2018/0008783 A1 offenbart einen Dosiermechanismus für einen Injektionspen. Beim Ausschütten wird eine Kupplungshülse axial nach distal verschoben relativ zu einer Antriebshülsse und elastische auslenkbare Arme greifen in Nuten im Pengehäuse ein und koppeln dadurch die Kupplungshülse rotationsfest zum Gehäuse.

US 2016/0045664 A1 offenbart einen wiederverwendbaren Injektionspen. Beim Dosiseinstellen wird eine Antriebshülse rotiert und wird so entlang einer Kolbenstange geschraubt. Zum Ausschütten wird ein Ausschütknopf gedrückt, wodurch eine Kupplungshülse rotiert zum Bewegen der Kolbenstange in Ausschüttrichtung, während die Antriebshülse rotationsfest zu einem inneren Gehäuseteil gekoppelt wird.

### DARSTELLUNG DER ERFINDUNG

Es ist eine Aufgabe der Erfindung mit einer Injektionsvorrichtung auf einfache Weise und mit wenigen Bauteilen ein Dosieren und Ausschütten zu ermöglichen.

Diese Aufgabe wird gelöst durch eine Injektionsvorrichtung und einen Dosier- und Ausschüttmechanismus gemäss den unabhängigen Ansprüchen. Bevorzugte Ausführungsformen sind Gegenstand der abhängigen Ansprüche.

Gemäss der Erfindung umfasst eine Injektionsvorrichtung, insbesondere eine Einweg-Injektionsvorrichtung, zum Ausschütten einer Dosis eines Produkts mittels manuellem Vorschub einer Kolbenstange in eine in der Injektionsvorrichtung gehaltenen Karpule einen Dosier- und Ausschüttmechanismus. Dieser Dosier- und Ausschüttmechanismus umfasst ein Gehäuse mit einer Längsachse, eine im Gehäuse längsverschiebbar angeordnete Antriebshülse zum Antreiben einer Kolbenstange, ein Betätigungselement zum Einstellen der Dosis, wobei das Betätigungselement rotationsfest mit der Antriebshülse verbunden ist und in Richtung Längsachse relativ zur Antriebshülse verschiebbar ist und wobei zum Einstellen der Dosis das Betätigungselement relativ zum Gehäuse drehbar ist. Dabei umfasst das Betätigungselement ein erstes Kopplungselement und das Gehäuse ein zweites Kopplungselement, wobei zum Ausschütten der Dosis das erste und das zweite Kopplungselement miteinander koppelbar sind, so dass im gekoppelten Zustand das Betätigungselement zusammen mit der Antriebshülse rotationsfest mit dem Gehäuse verbunden ist und in Richtung der Längsachse relativ zum Gehäuse verschiebbar ist bzw. verschiebbar bleibt.

Bei Einweg-Injektionsvorrichtungen bestehen andere Anforderungen als bei Mehrweg-Injektionsvorrichtungen. So muss beispielsweise bei Einweg-Injektionsvorrichtungen die Kolbenstange nicht zurücksetzbar sein, wenn die maximale Dosis aus der Karpule ausgeschüttet ist, da die Injektionsvorrichtung nach der letzten ausschüttbaren Dosis entsorgt wird. Dadurch ergeben sich neue Möglichkeiten bezüglich der Ausgestaltung des Einstell- und Ausschüttmechanismus.

Indem das Betätigungselement mittels seines ersten Kopplungselements direkt und ohne weiteres Zwischenelement mit dem Gehäuse mittels des im Gehäuse angeordneten zweiten Kopplungselements koppelbar ist, kann ein einfach konstruierbarer Dosier- und Ausschüttmechanismus geschaffen werden, welcher wenige Einzelteile benötigt. Das bedeutet, für die rotative Kopplung des Betätigungselements zum Ausschütten werden keine weiteren Bauteile benötigt, wie dies aus dem Stand der Technik üblich ist (siehe beispielsweise die oben genannte WO 2014/033195).

Zudem ermöglicht die beim Ausschütten rotativ feste aber verschiebbare Antriebshülse ein effektives und einfaches Antrieben der Kolbenstange. Die axiale Bewegung der Antriebshülse kann beispielsweise direkt in eine axiale Bewegung der Kolbenstange übertragen werden um diese in die Karpule hineinzubewegen, um das Produkt auszuschütten. Alternativ kann die axiale Bewegung übersetzt oder untersetzt werden, beispielsweise indem die Antriebshülse in Gewindeverbindung mit der Kolbenstange steht und diese in Rotation versetzt, so dass sich die Kolbenstange in distaler Richtung geschraubt wird. Je nach Gewindesteigung des Gewindes, mit dem die Kolbenstange im Gehäuse gelagert ist, kann mehr oder weniger Vorschub erzielt werden.

Die Injektionsvorrichtung umfasst eine Karpule, in der sich eine medizinische Substanz befindet. Um das Produkt aus der Karpule auszuschütten, kann mittels einer Kolbenstange der Injektionsvorrichtung beispielsweise ein Stopfen in der Karpule in einer Ausschüttrichtung in distaler Richtung verschoben. Vorzugsweise steht diese Ausschüttrichtung parallel zur Richtung der Längsachse des Gehäuses.

Der Dosier- und Ausschüttmechanismus umfasst ein Gehäuse, in welchem die Antriebshülse, die Kolbenstange und bevorzugt das Betätigungselement angeordnet sind. Dabei kann dieses Gehäuse gleichzeitig das äussere Gehäuse der Injektionsvorrichtung bilden oder die Injektionsvorrichtung kann alternativ eine zusätzliche eine Gehäusestruktur umfassen. In diesem Fall ist das Gehäuse des Dosier- und Ausschüttmechanismus vorzugsweise innerhalb der Gehäusestruktur der Injektionsvorrichtung angeordnet.

Ferner kann das Gehäuse des Dosier- und Ausschüttmechanismus innere Elemente wie beispielsweise einen Gehäuseeinsatz oder eine innere Gehäusehülse umfassen. Ein solcher Gehäuseeinsatz kann beispielsweise ein Dosisanzeigeelement und/oder die Antriebshülse drehbar lagern.

Um eine eingestellte Dosis auszuschütten wird durch eine manuell durch den Benutzer aufgebracht Kraft das Betätigungselement in Richtung der Längsachse relativ zum Gehäuse verschoben. Dabei wird auch die rotationsfest mit dem Betätigungselement verbunden Antriebshülse beim Ausschütten in Richtung Längsachse verschoben. Mittels der Antriebshülse wird die Kolbenstange angetrieben, so dass sie sich in die Karpule hineinbewegt und das Produkt aus der Karpule ausgeschüttet werden kann. Dabei bedeutet "manuell", dass keine sonstige Energie wie beispielsweise eine Kraft aus einer vorgespannten Feder zum Ausschütten verwendet wird. Der Vorschub der Kolbenstange zum Ausschütten des Produkts wird also nur durch den Benutzer aufgebracht. Die Kolbenstange kann durch die Antriebshülse gedreht werden, so dass sie sich beispielsweise durch eine Schraubbewegung in distaler Richtung bewegt. Alternativ kann die Kolbenstange durch die Antriebshülse nur verschoben werden und rotationsfest im Gehäuse geführt sein.

Das Betätigungselement ist zum Einstellen und Korrigieren einer Dosis rotierbar relativ zum Gehäuse. Zudem ist das Betätigungselement rotationsfest mit der Antriebshülse verbunden und in Richtung Längsachse relativ zur Antriebshülse verschiebbar. Dabei befindet sich vorzugsweise beim Einstellen einer Dosis das Betätigungselement in einer ersten axialen Position relativ zur Antriebshülse, in der die beiden Kopplungselement nicht miteinander gekoppelt sind.

Vorzugsweise dient das Betätigungselement auch zum Starten des Ausschüttvorgangs. Ein Ausschüttknopf ist also bevorzugt einteilig in das Betätigungselement integriert. Mit anderen Worten ausgedrückt, dient das Betätigungselement vorzugsweise sowohl als Dosiereinstellelement wie auch als Ausschüttknopf.

Beim Ausschütten der Dosis befindet sich das Betätigungselement bevorzugt in einer zweiten axialen Position relativ zur Antriebshülse, in der die beiden Kopplungselement miteinander gekoppelt sind, so dass das Betätigungselement und damit auch die Antriebshülse rotationsfest mit dem Gehäuse verbunden sind und in Richtung der Längsachse relativ zum Gehäuse verschiebbar sind. Dadurch lässt sich mit einer manuellen in distaler Richtung wirkenden Kraft, mit der das Betätigungselement und die Antriebshülse distal verschoben werden, mittels der Antriebshülse die Kolbenstange auf einfach Art und Weise zum Ausschütten der Dosis antrieben.

Das erste und das zweite Kopplungselement können wahlweise und lösbar miteinander gekoppelt werden, um in einem gekoppelten Zustand das Betätigungselement rotationsfest mit dem Gehäuse zu verbinden. Die Kopplungselemente können dabei in Form von Stift und Öffnung, Keil und Nut, ineinander greifenden Zahnräder ausgebildet oder über Kraftschluss zusammenwirkende Elemente ausgeführt sein. Dabei ist das erste Kopplungselement direkt im Betätigungselement, einteilig, mit dem Betätigungselement ausgebildet und das zweite Kopplungselement direkt im Gehäuse, einteilig mit dem Gehäuse, ausgebildet.

In der vorliegenden Beschreibung bezeichnet der Begriff "distal" eine zum vorderen, einstechseitigen Ende der Injektionsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Injektionsvorrichtung hin gerichtete Seite oder Richtung.

Der Begriff axial bezieht sich auf die Längsachse des Gehäuses. Entsprechend ist eine axiale Richtung parallel zur Längsachse des Gehäuses oder in Längsrichtung des Gehäuses. Eine radiale Richtung bezieht sich auf eine Richtung rechtwinklig zur Längsachse des Gehäuses.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP 1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Unter den Begriffen "Injektionsvorrichtung" oder "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektionssystem oder bei einem Injektor im Unterschied zu einem Infusionssystem die Injektionsnadel nicht über einen längeren Zeitraum von mehreren Stunden im Gewebe.

Unter "Einweg-Injektionsvorrichtung'", welche auch als "disposable" bezeichnet wird, ist eine Vorrichtung zu verstehen, welche nach einem oder mehreren Injektionsvorgängen jedoch spätestens nach der letzten ausschüttbaren Dosis entsorgt wird. Die Karpule ist bei einer solchen Einweg-Injektionsvorrichtung nicht auswechselbar. Demgegenüber kann bei Mehrweg-Injektionsvorrichtungen oder "reusable" Injektionsvorrichtungen die eingesetzte Karpule ausgetauscht werden, wenn das Produkt vollständig aus dieser ausgeschüttet worden ist. Bei solchen Mehrweg-Injektionsvorrichtungen wird beim Austausch der Karpule die Kolbenstange wieder in proximaler Richtung zurück ins Gehäuse geschoben, so dass diese wieder bereit zum Ausschütten des Produkts aus der neue Karpule ist.

Vorzugsweise ist das erste Kopplungselement ein flexibler Arm, welcher in radialer Richtung auslenkbar ist. Dieser kann mit dem zweiten Kopplungselement im Gehäuse zusammenwirken, beispielsweise in eine Vertiefung, Nut, Öffnung oder Auskragung im Gehäuse eingreifen. Der Arm ist bevorzugt elastisch in radialer Richtung auslenkbar oder verformbar und insbesondere elastisch krümmbar oder verbiegbar. Dadurch kann rasch und einfach eine rotative Kopplung zwischen dem Betätigungselement und dem Gehäuse hergestellt werden. Alternativ dazu kann der Arm zwar flexibel auslenkbar sein, aber nicht elastisch ausgebildet sein. In diesem Fall muss der Arm von einer manuell aufgebrachten Kraft von einer Ausgangsposition in eine ausgelenkten Position bzw. durch die manuelle Kraft von der ausgelenkten Position in die Ausgangsposition zurückbewegt werden. Diese Auslenkung kann über eine Getriebefläche ermöglicht sein.

Der Arm ist vorzugsweise auf einer Aussenfläche des Betätigungselements angeordnet. Weiter umfasst in einer bevorzugten Ausführung das Betätigungselement auf seiner Aussenseite mindestens zwei flexible Arme, welche je mit einem zweiten Kopplungselement im Gehäuse zusammenwirken können. Dadurch kann die Kopplung verstärkt werden.

Alternativ besteht auch die Möglichkeit, dass das erste Kopplungselement nicht als Arm sondern als Nocken, starre Auskragung, Vertiefung oder Nut ausgebildet ist.

Bevorzugt umfasst der Dosier- und Ausschüttmechanismus ein Verriegelungselement, mit dem der flexible Arm in radialer Richtung blockierbar ist. Nachdem der Arm mit dem zweiten Kopplungselement in Eingriff gebracht worden ist, kann mindestens ein Bereich des Arms mittels des Verriegelungselements blockiert oder fixiert werden, so dass der Arm sich nicht mehr radial bewegen kann. Dadurch kann sich die Kopplung zwischen Betätigungselement und Gehäuse nicht unbeabsichtigt lösen. Das Verriegelungselement kann beispielsweise als Arm, Schieber, Sicherungsmutter, Schnappelement oder als Hülse ausgebildet sein. Vorzugsweise ist das Verriegelungselement an der Antriebshülse angeordnet und insbesondere einteilig mit dieser verbunden. Das ermöglicht eine kompakte Konstruktion.

In einer bevorzugten Ausführung ist durch eine Verschiebung des Betätigungselements in Richtung Längsachse relativ zur Antriebshülse der flexible Arm mit dem Verriegelungselement blockierbar. Falls das Verriegelungselement nocken- oder ringförmig ausgebildet ist, kann dieses in radialer Richtung beispielsweise unter den flexiblen Arm verschoben werden, wenn sich dieser in Eingriff mit dem zweiten Kopplungselement befindet, um so eine radialen Bewegung des Arms zum Zentrum der Injektionsvorrichtung hin zu blockieren. Vorzugsweise wird, wenn der Benutzer das Betätigungselement in distaler Richtung bewegt, um den Ausschüttvorgang zu starten, das Betätigungselement relativ zur Antriebshülse verschoben. Dabei wird vorzugsweise der mit dem zweiten Kopplungselement in Eingriff stehende Arm mit dem Verriegelungselement blockiert, so dass sich der Arm nicht aus dem Eingriff lösen kann. Dadurch wird eine einfache und sichere rotative Kopplung des Betätigungselements (und damit auch der Antriebshülse) mit dem Gehäuse erreicht.

Alternativ dazu besteht auch die Möglichkeit, dass durch eine Drehbewegung des Betätigungselements relativ zum Gehäuse der flexible Arm mit dem Verriegelungselement blockiert werden kann, beispielsweise indem eine Sicherungsmutter über den Arm geschraubt wird.

Vorzugsweise ist das zweite Kopplungselement eine Nut, in der mindestens ein Bereich des ersten Kopplungselements aufgenommen werden kann. Die Nut ist bevorzugt in Richtung Längsachse im Gehäuse ausgebildet. Die Nut ist einfach herstellbar und ermöglicht eine rasche Kopplung mit dem ersten Kopplungselement. Weiter weist das Gehäuse vorzugsweise mehrere über den Umfang verteilte Nuten auf, so dass das erste Kopplungselement an unterschiedlichen rotativen Positionen mit jeweils einer Nut in Eingriff gebracht werden kann.

In einer bevorzugten Ausführung dient die Nut sowohl als Kopplungselement wie auch als Klickelement zum Erzeugen eines akustischen und/oder taktilen Signales beim Einstellen und Korrigieren einer Dosis. Dieses wird erzeugt indem das erste Kopplungselement über die Nut bewegt wird. Das erste Kopplungselement greift dann nur kurzeitig in die Nut und federt anschliessend zurück, wodurch das akustische und/oder taktile Signal erzeugt wird.

Alternativ besteht auch die Möglichkeit, dass das zweite Kopplungselement nicht als Nut sondern beispielsweise als Öffnung, Haken, Rippe oder Zahnelement ausgebildet ist.

In einer bevorzugten Ausführung, bei der das erste Kopplungselement als Arm und das zweite Kopplungselement als Nut ausgebildet sind, kann vorzugsweise beim Einstellen und Korrigieren einer Dosis ein freies Ende des flexiblen Arms mit der Nut derart zusammenwirken, dass ein taktiles und/oder akustisches Signal erzeugt werden kann. Das Signal in der Form eines Klicks oder/und einer Vibration ist erzeugbar, wenn das freie Ende des flexiblen Arms über die Nut, insbesondere quer zur Nut bewegt wird und dabei elastisch auslenkt in diese zurückfedert, insbesondere wenn das Betätigungselement beim Einstellen und Korrigieren einer Dosis relativ zum Gehäuse rotiert wird. Die Nut dient also einerseits als Signalerzeugungselement und anderseits als Kopplungselement. Um die Kopplung zu sichern, umfasst in dieser Ausführung die Antriebshülse vorzugsweise ein Verriegelungselement, welches zum Arm bewegbar ist und den Arm blockieren kann, indem durch das Verriegelungselement mindestens ein Bereich des Arms in der Nute gehalten wird und dadurch eine Rotation des Betätigungselements relativ zum Gehäuse blockiert.

Bevorzugt umfasst das Gehäuse mehrere Nuten, so dass bei einer Bewegung des Arms über die Nuten mehrere Klicks und Vibrationen erzeugt werden können.

In einer bevorzugten Ausführung ist eine nicht selbsthemmende Gewindeverbindung zwischen Antriebshülse und Kolbenstange vorhanden, so dass durch eine Verschiebung der Antriebshülse axialkraftübertragend relativ zum Gehäuse die Kolbenstange in Rotation versetzt werden kann, um die Dosis auszuschütten. In dieser Ausführung steht die Kolbenstange zudem in Gewindeverbindung mit dem Gehäuse. So wird die durch den Benutzer aufgebrachte Kraft durch die axiale Verschiebung der Antriebshülse in distaler Richtung auf die Kolbenstange übertragen, wodurch die Kolbenstange durch das Gewinde im Gehäuse geschraubt wird und in distaler Richtung in die Karpule hineinbewegt werden kann.

Dabei weist die Kolbenstange vorzugsweise ein erstes Gewinde, mit dem die Kolbenstange im Gehäuse eingeschraubt ist, und ein zweites Gewinde auf, welches eine zum ersten Gewinde unterschiedliche Steigungsrichtung aufweist. Die Antriebshülse steht vorzugsweise mit dem zweiten Gewinde in Gewindeverbindung. So kann beim Einstellen und Korrigieren einer Dosis die Antriebshülse auf dem zweiten Gewinde der nicht rotierenden Kolbenstange auf und ab bewegt werden, während beim Ausschütten der Dosis die nicht rotierende Antriebshülse durch die axiale Verschiebung die Kolbenstange in Rotation versetzt und diese mit dem ersten Gewinde durch das Gehäuse geschraubt wird. Vorzugsweise weisen das erste und das zweite Gewinde nicht nur eine unterschiedliche Richtung (Drehsinn) auf, sondern haben auch eine unterschiedliche Steigung. In diesem Fall kann eine Übersetzung bzw. Untersetzung zwischen der Bewegung der Antriebshülse und dem Vorschub der Kolbenstange realisiert werden. Das erste und das zweite Gewinde sind je auf einem ersten und einem zweiten in axialer Richtung sich erstreckenden Gewindeabschnitt auf der Kolbenstange aufgebracht, wobei sich der erste und der zweite Gewindeabschnitt nicht, teilweise oder vollständig überlappen. Das erste, das zweite, beide oder keines der Gewinde können selbsthemmend ausgeführt sein.

Vorzugsweise umfasst das Gehäuse des Dosier- und Ausschüttmechanismus im Innern eine gehäusefeste Gehäusehülse, wobei das zweite Kopplungselement auf der Innenseite der Gehäusehülse ausgebildet ist. Das Gehäuse umfasst somit eine äussere Gehäusestruktur oder äussere Gehäusehülse und eine im Innern angeordnete innere Gehäusehülse. Dabei bedeutet "gehäusefest", dass die innere Gehäusehülse rotationsfest und axialfest unlösbar mit der äusseren Gehäusehülse verbunden ist. Die innere Gehäusehülse ermöglicht eine vorteilhafte Anordnung der Bauteile im Innern der äusseren Gehäusehülse, da Bauteile im Innern an der inneren Gehäusehülse abgestützt bzw. mit dieser fest, bewegbar oder lösbar verbunden werden können. Weiter können radial zwischen einer Innenseite der äusseren Gehäusehülse und einer Aussenseite der inneren Gehäusehülse auf der inneren Gehäusehülse Bauteile gelagert werden. Dies ist insbesondere für rotierbare Elemente vorteilhaft, da für die Rotation auf der Aussenseite der inneren Gehäusehülse geringere Reibmomente entstehen als bei einer Rotation auf der Innenseite des äusseren Gehäusehülse, da der das Drehmoment bestimmende Radius der inneren Gehäusehülse kleiner ist, als der Radius der äusseren Gehäusehülse.

Die innere Gehäusehülse ist dabei bevorzugt koaxial zur Längsachse angeordnet und an einem distalen Ende mit der äusseren Gehäusehülse unlösbar verbunden. Die äussere Gehäusehülse kann mit der inneren Gehäusehülse einteilig ausgeführt sein oder die äussere Gehäusehülse und die innere Gehäusehülse können als Einzelteile hergestellt werden und anschliessend unlösbar miteinander axial- und rotationsfest verbunden werden, beispielsweise durch Verschweissen oder Verkleben.

Bevorzugt umfasst der Dosier- und Ausschüttmechanismus eine im Gehäuse angeordnete Dosisanzeigehülse zum Anzeigen einer eingestellten Dosis, wobei die Dosisanzeigehülse beim Einstellen einer Dosis mit dem Betätigungselement mittels eines Kupplungsmechanismus rotationsfest gekoppelt ist. Hierzu können beispielsweise das Betätigungselement und die Dosierhülse je Zähne umfassen, welche ineinander eingreifen können, um das Betätigungselement mit der Dosierhülse rotationsfest zu koppeln. Alternativ dazu kann der Kupplungsmechanismus einen Keil und eine entsprechende Nut umfassen oder der Kupplungsmechanismus umfasst Elemente welche kraftschlüssig miteinander koppelbar sind. In allen Ausführungen erlaubt der Kupplungsmechanismus das wahlweise rotative Koppeln des Betätigungselements mit der Dosisanzeigehülse oder das Lösen voneinander.

Dabei wird die Dosisanzeigehülse nicht durch den Benutzer betätigt und muss daher keinen Griff oder sonstige Betätigungselemente aufweisen, was eine kompakte Konstruktion ermöglicht. Die Dosisanzeigehülse wird beim Einstellen und Korrigieren einer Dosis mittels des Betätigungselements relativ zum Gehäuse rotiert, verschoben oder schraubenförmig bewegt. Zum Anzeigen der Dosis kann die Dosisanzeigehülse beispielsweise auf ihrer Aussenseite Zahlen aufweisen.

Vorzugsweise ist beim Dosier- und Ausschüttmechanismus beim Ausschütten die Dosisanzeigehülse mittels des Kupplungsmechanismus entkoppelt, so dass die Dosisanzeigehülse relativ zum Betätigungselement rotierbar ist. Falls der Kupplungsmechanismus Zähne oder Rippen umfasst, sind diese vorzugsweise in Richtung Längsachse ausgerichtet. Dadurch können zum Ausschütten mittels einer relativen Verschiebung des Betätigungselements zur Dosisanzeigehülse die Zähne des Betätigungselements aus Zähnen oder Nuten der Dosisanzeigehülse heraus geschoben werden, wodurch sich die Kopplung aufhebt und die Dosisanzeigehülse relativ zum Betätigungselement rotierbar ist. Durch die Entkopplung von Dosisanzeigehülse und Betätigungselement kann die Dosisanzeigehülse beispielsweise in das Gehäuse eingeschoben oder eingeschraubt werden, während das Betätigungselement rein axial relativ zum Gehäuse verschiebbar ist zum Antreiben der Kolbenstange. Die Dosisanzeigehülse kann verschiebbar, rotierbar oder mittels eines Gewindes am Gehäuse oder einem allenfalls vorhandenen inneren Gehäuseeinsatz oder inneren Gehäusehülse bzw. in einem durch koaxial angeordnete Bauteile gebildeten Ringspalt gelagert sein.

Bevorzugt umfasst die Dosisanzeigehülse ein radiales Klickelement, welches mit dem Betätigungselement zusammenwirken kann, um ein taktiles und/oder akustisches Signal zu erzeugen. Das Signal wird dabei vorzugsweise beim Ausschütten erzeugt, wenn das Betätigungselement relativ zur Dosisanzeigehülse rotiert. Das Betätigungselement weist bevorzugt Gegenelemente in Form von Stegen, Nuten oder Auskragungen auf, so dass wenn das Klickelement, vorzugsweise in Form eines flexiblen radialen Arms oder einer flexiblen Lasche, beim Ausschütten über die Gegenelemente bewegt wird, das taktile und/oder akustische Signal in Form eines Klicks oder einer Vibration erzeugt wird. Dieses Signal gibt dem Benutzer eine Rückmeldung beim Ausschütten der Dosis. Die Gegenelemente können Teil des oben beschriebenen Kupplungsmechanismus am Betätigungselement sein.

In einer bevorzugten Ausführung steht die Dosisanzeigehülse mit einem innenliegenden Gewinde mit einem Aussengewinde der inneren Gehäusehülse in Gewindeverbindung. Dadurch kann auf einer Aussenseite der Dosisanzeigehülse eine Zahlenskala für die Angabe der Dosis angeordnet werden, ohne dass ein Gewinde auf der Aussenseite der Dosisanzeigehülse vonnöten ist, welches die Platzierung der Skala beschränken würde. Die Dosisanzeigehülse wird vorzugsweise zusammen mit dem Betätigungselement beim Einstellen bzw. Korrigieren einer Dosis in proximaler Richtung aus dem bzw. in das Gehäuse geschraubt. Beim Ausschütten der Dosis ist die Dosisanzeigehülse bevorzugt vom Betätigungselement entkoppelt und wird in distaler Richtung zurück ins Gehäuse geschraubt, während das Betätigungselement rein axial nach distal verschoben werden kann.

Vorzugsweise weist die innere Gehäusehülse auf ihrer Aussenseite eine distale und eine proximale Rippe auf, wobei die distale Rippe an ein Anschlagelement auf der Innenseite der Dosisanzeigehülse anschlägt um eine minimal einstellbare Dosis zu begrenzen bzw. das Ende einer Ausschüttung zu definieren und die proximale Rippe an ein Anschlagelement anschlägt, um eine maximal einstellbare Dosis zu begrenzen. Dabei kann der Anschlag in radialer oder in axialer Richtung ausgebildet sein.

In einer bevorzugten Ausführung umfasst die Antriebshülse an einem proximalen Ende ein fest und unlösbar mit der Antriebshülse verbundenes elastisches Element, wobei das Betätigungselement mit einer durch das elastische Element erzeugten Vorspannkraft in proximaler Richtung beaufschlagbar ist. Das elastische Element ist beispielsweise eine Feder aus Metall oder Kunststoff oder ein elastisch verformbares Material wie ein Elastomer. Das elastische Element ist dabei unlösbar mit der Antriebshülse verbunden und/oder zwischen Antriebshülse und Betätigungselement gehalten.

Durch die Vorspannkraft kann das Betätigungselement relativ zur Antriebshülse in einer proximalen Position gehalten werden. Das ist vorteilhaft, wenn beispielsweise das Betätigungselement in einer relativ zur Antriebshülse proximalen Position vom Gehäuse rotativ entkoppelt oder rotativ gekoppelt bleiben soll. In diesem Fall kann mittels der Vorspannkraft eine ungewollte Kopplung bzw. eine ungewollte Kopplung des Betätigungselements mit dem Gehäuse vermieden werden. Erst mittels einer vom Benutzer aufgebrachten manuellen distal wirkenden Kraft, welche die Vorspannkraft übersteigt, kann das Betätigungselement relativ zur Antriebshülse in distaler Richtung verschoben werden, um das erste und zweite Kopplungselement miteinander zu koppeln bzw. zu entkoppeln.

Bevorzugt sind dabei das elastische Element und die Antriebshülse einteilig ausgebildet. So kann das elastische Element beispielsweise als Spiralfeder oder Wellenfeder ausgebildet sein, welche sich an einem zylindrischen Körper der Antriebshülse am proximalen Ende anschliesst, beispielsweise an diesen mittels Kunststoffspritzgussverfahren aufgespritzt ist. Weiter kann das elastische Element aus einem Elastomermaterial gebildet sein und mittels Mehrkomponenten-Spritzguss einteilig mit der Antriebshülse, welche beispielsweise aus einem Thermoplast besteht, hergestellt werden. Eine solche einteilige Antriebshülse reduziert die Anzahl Bestandteil der Injektionsvorrichtung und vereinfacht die Montage.

Alternativ dazu besteht auch die Möglichkeit, dass das elastische Element separat von der Antriebshülse als Einzelteil ausgebildet ist, beispielsweise als Metall oder Kunststofffeder in der Form einer Spiral- oder Wellenfeder.

Vorzugsweise umfasst der Dosier- und Ausschüttmechanismus eine Stoppmutter zur Begrenzung einer letzten Dosis. Die Stoppmutter steht in Gewindeverbindung mit einem Aussengewinde der Antriebshülse und wirkt mit dem zweiten Kopplungselement zusammen. Das zweite Kopplungselement führt die Stoppmutter in Richtung Längsachse und verhindert eine Rotation der Stoppmutter relativ zum Gehäuse. Die Stoppmutter schlägt an einen Anschlag der Antriebshülse an, wenn die letzte ausschüttbare Dosis eingestellt wurde und verhindert so, dass eine letzte Dosis einstellbar ist, welche die Kapazität der Karpule übersteigen würde.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt in perspektivischer Ansicht eine Explosionsdarstellung der Einzelteile des erfindungsgemässen Injektors mit dem Dosier- und Ausschüttmechanismus;
- Fig. 2: zeigt eine Schnittansicht des Injektors in einer Ausgangsstellung, wobei der Schnitt durch die Längsachse des Injektors verläuft;
- Fig. 3a: zeigt eine perspektivische Ansicht eines Betätigungselements;
- Fig. 3b: zeigt eine perspektivische Ansicht des proximalen Bereichs des Injektors während dem Einstellen und Korrigieren einer Dosis, wobei der distale Bereich weggeschnitten ist und der Schnitt rechtwinklig zur Längsachse des Injektors verläuft;
- Fig. 4a: zeigt einen mittleren Bereich des Injektors in einer Schnittansicht beim Einstellen und Korrigieren einer Dosis;
- Fig. 4b: zeigt den mittleren Bereich aus Figur 4a beim Ausschütten einer Dosis und
- Fig. 5: zeigt eine perspektivische Schnittansicht des proximalen Bereichs des Injektors beim Ausschütten einer Dosis, wobei der Schnitt durch die Längsachse verläuft.

### FIGURENBESCHREIBUNG

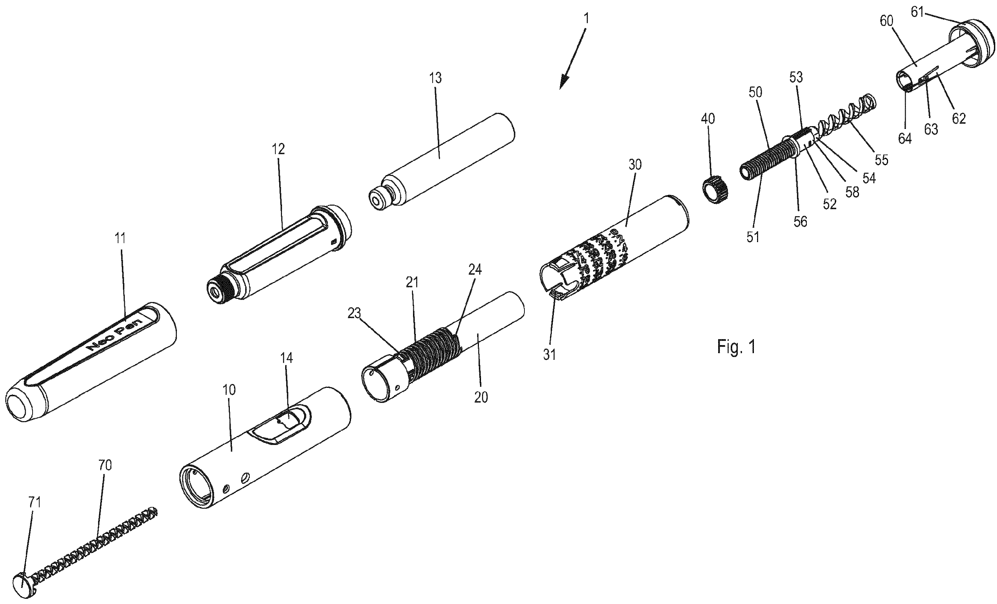

Figur 1 zeigt eine perspektivische Ansicht der Einzelteile eines erfindungsgemässen Injektors 1 mit einem Dosier- und Ausschüttmechanismus in einer Explosionsdarstellung. Dabei befindet sich das distale, einstechseitige Ende des Injektors 1 im linken, unteren Bereich der Figur 1 und das proximale Ende des Injektors 1 im rechten, oberen Bereich der Figur 1.

Der Injektor 1 ist in der gezeigten Ausführung als Einweginjektor ausgebildet. Wie in Figur 1 ersichtlich, umfasst der Injektor 1 eine abnehmbare Schutzkappe 11, ein längliches, zylinderförmiges Gehäuse 10, welches zugleich das Gehäuse eines Dosier- und Ausschüttmechanismus bildet und einen Karpulenhalter 12 in dem eine mit einer medizinischen Substanz gefüllte Karpule 13 gehalten ist. Weiter umfasst der Injektor 1 den Dosier- und Ausschüttmechanismus. Dieser beinhaltet das Gehäuse 10, welches eine äussere Gehäusehülse und eine im Innern der äusseren Gehäusehülse angeordnet innere Gehäusehülse 20 umfasst, eine Dosisanzeigehülse 30, eine Antriebshülse 50, eine Stoppmutter 40, welche in Gewindeverbindung mit der Antriebshülse 50 steht, ein Betätigungselement 60 zum Einstellen und Korrigieren einer Dosis und zum Auslösen des Ausschüttvorgangs und eine innerhalb der Antriebshülse 50 angeordnete Kolbenstange 70, welche durch die Antriebshülse 50 angetrieben werden kann zum Ausschütten der medizinischen Substanz aus der Karpule 13.

Nachfolgend wird auf die strukturellen Merkmale der einzelnen Bestandteile des Injektors 1 im Detail eingegangen. Die Funktion, insbesondere das Einstellen, Korrigieren und Ausschütten einer Dosis, ist im Anschluss beschrieben.

Der Karpulenhalter 12 ist an einem distalen Ende des Gehäuses 10 mittels einer Schnappverbindung rotationsfest und axialfest mit dem Gehäuse 10 verschnappbar bzw. verschnappt. Alternativ kann der Karpulenhalter 12 auch mit der inneren Gehäusehülse 20 verschnappbar ausgebildet sein. Der Karpulenhalter 12 lagert die Karpule 13 und weist an seinem distalen Ende ein Verbindungselement auf, an dem eine Injektionsnadel oder Kanüle (nicht dargestellt) angebracht werden kann.

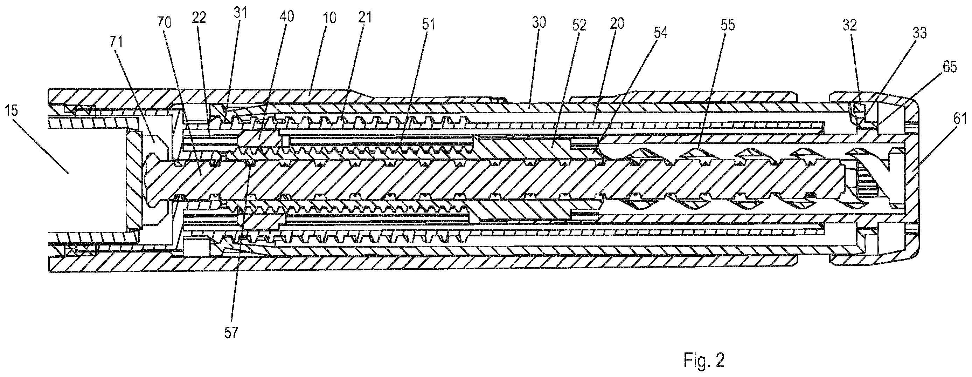

Die zylindrisch und hülsenförmig ausgebildete äussere Gehäusehülse des Gehäuses 10 weist auf dem Zylindermantel eine Öffnung 14 auf, durch welche die Dosisanzeigehülse 30 ersichtlich ist. Die innere Gehäusehülse 20 hat ebenfalls eine zylindrische Form und ist koaxial zur äusseren Gehäusehülse angeordnet. An einem distalen Bereich ist die innere Gehäusehülse 20 sowohl in axialer Richtung wie auch rotativ unbeweglich relativ zur äusseren Gehäusehülse mit dieser verbindbar oder verbunden. Im distalen Bereich weist die innere Gehäusehülse 20 in ihrem Innern ein Innengewinde auf, in welchem die Kolbenstange 70 einschraubbar oder eingeschraubt ist, ersichtlich in Figur 2. Die Figur 2 zeigt eine Schnittansicht des Injektors, wobei der Schnitt durch die Längsachse verläuft. Auf der Aussenseite weist die innere Gehäusehülse 20 ein Aussengewinde 21 auf, welches sich auf einem distalen Abschnitt etwa über die halbe axiale Länge der inneren Gehäusehülse 20 erstreckt. Mit dem Aussengewinde steht die Dosisanzeigehülse 30 in Gewindeverbindung. Im Innern weist die innere Gehäusehülse 20 mehrere über den Umfang verteilte in Richtung Längsachse ausgerichtete Längsnuten 22 auf, welche als Kopplungselement dienen und sich über die gesamte Länge der inneren Gehäusehülse 20 erstrecken, erkennbar in Figur 2.

Wie in Figur 1 ersichtlich, weist am Anfang und am Ende des Aussengewindes 21 die innere Gehäusehülse 20 je einen radialen Steg 23, 24 auf. Diese dienen als Anschläge und wirken mit einer radialen Auskragung 31 im Innern der Dosisanzeigehülse 30 zusammen und begrenzt so eine minimal bzw. eine maximal einstellbare Dosis.

Im distalen Endbereich weist die innere Gehäusehülse 20 einen zylindrischen Abschnitt mit einem grösseren Durchmesser auf, welcher fest mit der äusseren Gehäusehülse verbunden ist. Im Innern dieses distalen Endbereichs umfasst die innere Gehäusehülse 20 über den Umfang verteilt radial zum Zentrum hin ragende Rippen (nicht dargestellt). Die Rippen weisen in axialer Richtung gesehen eine Steigung auf, wobei die Steigung in proximaler Richtung zunimmt. Mit anderen Worten ausgedrückt, sind die Rippen axial keilförmig ausgebildet und weisen distal eine kleinere radiale Höhe auf als proximal, wobei die radiale Höhe stetig Richtung proximal zunimmt (Steigung). Jedoch können die Rippen in einer alternativen Ausführung auch ohne Steigung ausgebildet sein. Die Rippen sind in jeder Ausführung so dimensioniert bzw. materialisiert, dass sie plastisch verformbar sind. Bei der Montage wird die Karpule 13 zuerst in den Karpulenhalter 12 eingeführt. Anschliessend wird der Karpulenhalter 12 mit dem distalen Endbereich der inneren Gehäusehülse 20 verschnappt. Beim Zusammenführen des Karpulenhalters 12 mit der inneren Gehäusehülse 20 berührt zuerst die im Karpulenhalter 12 befindliche Karpule 13 bzw. deren Flammrand die Rippen der Gehäusehülse 20 an einer zum Zentrum weisenden Kante. Wird der Karpulenhalter 12 weiter in proximale Richtung in die innere Gehäusehülse 20 hineingeschoben, deformieren sich die Rippen plastisch. Das bedeutet, die Rippen werden durch die Karpule 13 bleibend verformt. Dabei werden die Rippen entweder als ganzes aus ihrer ursprünglichen Position zur Seite gedrückt oder die Rippen nehmen zumindest teilweise die Form der Aussenkontur der Karpule 13 an. Wenn der Karpulenhalter 12 nun mit der inneren Gehäusehülse 20 verschnappt wird, ist die Karpule 13 axial wie auch radial spielfrei und unbeweglich im Karpulenhalter 12 gehalten, da der Karpulenhalter 12 eine Spannkraft in proximaler Richtung auf die Karpule 13 ausübt und diese dadurch auf die verformten Rippen drückt.

Die Dosisanzeigehülse 30 steht, wie erwähnt, in Gewindeverbindung mit dem Aussengewinde 21 der inneren Gehäusehülse 20. Die Dosisanzeigehülse 30 ist somit in einem Ringspalt über der inneren Gehäusehülse 20 und innerhalb der äusseren Gehäusehülse angeordnet. Ferner hat die Dosisanzeigehülse 30 die Form eines Hohlzylinders. Sie weist in einem distalen Bereich auf ihrer Aussenseite eine umlaufende Zahlenskala auf, welche durch die Öffnung 14 in der äusseren Gehäusehülse von aussen ablesbar ist und dem Benutzer die eingestellte Dosis anzeigt.

Am distalen Ende umfasst die Dosisanzeigehülse 30 zwei radiale Auskragungen 31 auf der Innenseite, erkennbar in Figur 1. Diese sind auf einer Lasche oder einem Schnapper oder alternativ direkt auf der Innenseite angebracht, so dass die Dosisanzeigehülse 30 bei der Montage über den Steg 24 mit den Auskragungen 31 in das Aussengewinde 21 der Gehäusehülse 20 eingeschnappt werden kann. Im montierten Zustand laufen die Auskragungen 31 somit im Aussengewinde 21, wodurch die Dosisanzeigehülse 30 in Gewindeverbindung mit der Gehäusehülse 20 steht. In der vollständig eingeschraubten Position der Dosisanzeigehülse 30 schlägt eine Auskragung 31 am Steg 23 der Gehäusehülse 20 an, so dass die minimale Dosis bzw. die Schraubbewegung der Dosisanzeigehülse 30 in das Gehäuse hinein durch den Steg 23 begrenzt wird. Die maximale Dosis bzw. die Schraubbewegung der Dosisanzeigehülse 30 aus dem Gehäuse 10 heraus wird durch den Steg 24 begrenzt.

Wie in Figur 2 ersichtlich, weist die Dosisanzeigehülse am proximalen Ende 30 eine Verjüngung oder einen Abschnitt mit einem kleineren Innendurchmesser auf. Auf der Innenseite in diesem Abschnitt sind radial zum Zentrum nach innen weisende Zähne 33 ausgebildet, welche lösbar mit Zähne 65 des Betätigungselements 60 in Eingriff gebracht werden können. Diese lösbare Verzahnung zwischen Dosisanzeigehülse 30 und Betätigungselement 60 bildet einen Kupplungsmechanismus. Zudem weist die Dosisanzeigehülse 30 in diesem Abschnitt einen flexiblen, radialen Klickarm 32 auf. Dieser kann, wenn er über Zähnen oder Nuten des Betätigungselements 60 bewegt wird, ein akustisches Klickgeräusch erzeugen.

Im Innern der inneren Gehäusehülse 20 befindet sich die Antriebshülse 50. Diese ist im Wesentlichen zylinderförmig und weist einen starren und einen elastisches Abschnitt auf. Der starre Abschnitt ist wiederum in einen distalen Bereich 51 mit einem Aussengewinde und in einen proximalen Bereich 52 mit einem Verriegelungselement 58 unterteilt. Auf dem Aussengewinde ist die Stoppmutter 40 aufgeschraubt.

Ferner umfasst die Antriebshülse 50 im mittleren Bereich eine umlaufende Wandung 56, welche den starren Abschnitt in den distalen Bereich 51 und den proximalen Bereich 52 unterteilt. Der proximale Bereich 52 umfasst zwei über dem Umfange einander gegenüber liegende Führungen 53 in Form von axialen Stegen, welche in axiale Nuten 64 im Betätigungselement eingreifen. Der proximale Bereich 52 bildet zudem mit einem zylindrischen Bereich ein Verriegelungselement 58, um eine radiale Auslenkung von Armen 62 des Betätigungselements 60 zu blockieren. Dies ist weiter unten in Zusammenhang mit dem Ausschüttvorgang im Detail beschrieben. Am proximalen Ende des starren proximalen Bereichs 52 weist dieser einen abgesetzten Bereich 54 auf, welcher einen kleineren Aussendurchmesser hat als der übrige Bereich. Der elastische Abschnitt der Antriebshülse 50 ist als Kunststoffspiralfeder 55 ausgebildet. Das bedeutet, die Spiralfeder 55 aus Kunststoff ist am proximalen Ende des starren Abschnitts aufgespritzt und fest bzw. einstückig mit diesem verbunden. Im eingebauten Zustand befindet sich die Spiralfeder 55 somit zwischen dem proximalen Ende des proximalen Bereichs 52 der Antriebshülse 50 und einer Innenwandung des Betätigungselements 60. Die Spiralfeder 55 ist dabei in axialer Richtung leicht komprimiert, so dass das Betätigungselement 60 mit einer durch die Vorspannung erzeugten Kraft in proximaler Richtung beaufschlagt ist.

In einer nicht erfindungsgemäßen Alternative kann die Feder aus Metall oder Kunststoff auch getrennt von der Antriebshülse 50 ausgebildet sein. Sie ist in diesem Fall distal am proximalen Bereich 52 und proximal an der Innenwandung des Betätigungselements 60 abgestützt.

Im Inneren der Antriebshülse 50 befindet sich koaxial angeordnet die Kolbenstange 70. Die Antriebshülse 50 weist im Inneren radiale Eingriffstege 57 auf, welche in ein Linksgewinde der Kolbenstange 70 eingreifen. Die Antriebshülse 50 ist somit drehbar relativ zur Kolbenstange 70 wie auch zum Gehäuse 10 gelagert.

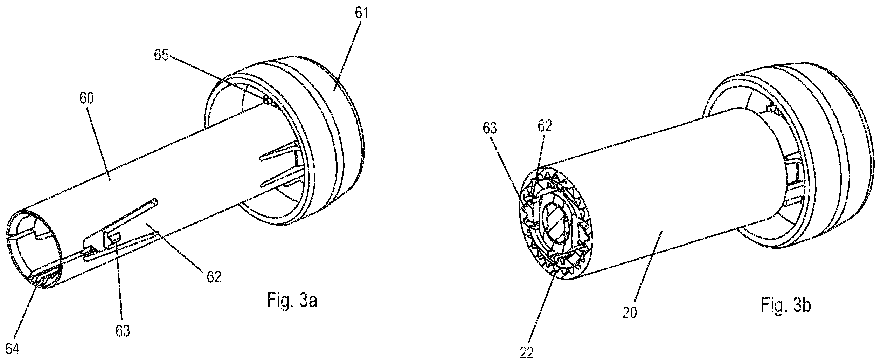

Mit der Antriebshülse 50 ist rotationsfest und axial verschiebbar das Betätigungselement 30 verbunden. Dieses ist einzeln in Figur 3a dargestellt. Es umfasst einen zylindrischen Körper, an den im proximalen Bereich ein Knopfelement 61 anschliesst. Der zylindrische Körper weist eine axiale Öffnung oder Bohrung auf in der sich auf der Innenseite die axialen Nuten 64 befinden. In diese greifen die axialen Stege der Führung 53 der Antriebshülse 50 ein und koppeln so das Betätigungselement 60 rotationsfest aber axial verschiebbar mit der Antriebshülse 50. Wie am besten in Figur 3a erkennbar, sind in Umfangrichtung einander um 180° versetzt in der Wandung des zylindrischen Körpers Kopplungselemente in Form von flexiblen Armen 62 ausgebildet. Diese Arme 62 sind in radialer Richtung elastisch auslenkbar. Zudem weisen die Arme 62 je auf der Aussenseite an ihrem freien Ende einen Nocken 63 auf.

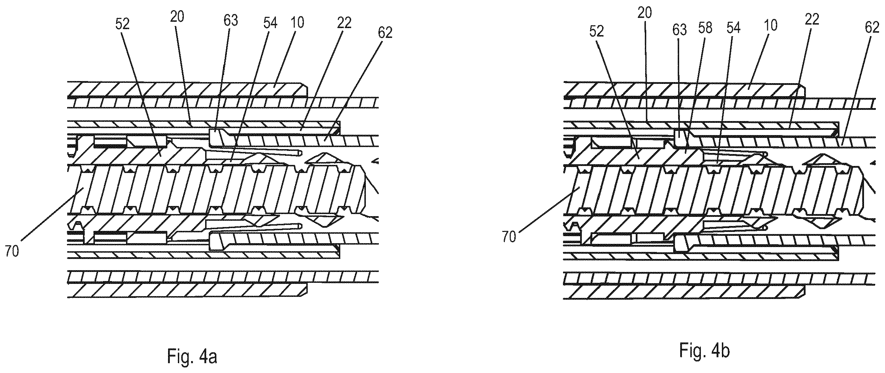

In Figur 3b ist in perspektivischer Ansicht der proximale Bereich des Injektors gezeigt, wobei der distale Bereich weggeschnitten ist und der Schnitt rechtwinklig zur Längsachse verläuft. In dieser Figur wie auch in Figur 4a ist ersichtlich, dass die Nocken 63 der Arme 62 in die Längsnuten 22 der inneren Gehäusehülse 20 hineinragen. In einem proximalen Bereich des zylindrischen Körpers des Betätigungselements 60 weist dieser umlaufend angeordnete Zähne 65 auf, welche mit den Zähnen 33 der Dosisanzeigehülse 30 in Eingriff gebracht werden können, um das Betätigungselement 60 rotativ mit der Dosisanzeigehülse 30 zu koppeln.

Das Knopfelement 61 umfasst einen Mantel, welcher über den proximalen Bereich des zylindrischen Körpers ragt. Mit anderen Worten ausgedrückt, ist das Knopfelement 61 pilzförmig ausgestaltet.

Zudem ist das Knopfelement 61 einteilig mit dem zylindrischen Körper des Betätigungselements 60 ausgebildet und somit axial- und rotationsfest mit dem zylindrischen Körper verbunden.

Wie erwähnt und in Figur 2 ersichtlich, ist auf dem Aussengewinde 21 der inneren Gehäusehülse 20 die Stoppmutter 40 aufgeschraubt. Diese hat auf ihrer Aussenseite axiale ausgerichtete Stege, welche in die Längsnuten 22 auf der Innenseite der Gehäusehülse 20 eingreifen. Die Stoppmutter 40 ist demnach drehbar relativ zur Antriebshülse 50 und axial verschiebbar aber rotationsfest relativ zur Gehäusehülse 20 gelagert.

Die Kolbenstange 70 umfasst ein linksdrehendes Gewinde (Linksgewinde) und ein rechtsdrehendes Gewinde (Rechtsgewinde). Mit dem Rechtsgewinde ist die Kolbenstange 70 in die innere Gehäusehülse 20 eingeschraubt. Mit dem Linksgewinde steht die Antriebshülse 50 in Gewindeeingriff. Dabei sind die Gewindesteigungen vorzugsweise unterschiedlich. So kann beispielsweise das Rechtsgewinde eine doppelt so grosse Steigung aufweisen, wie das Linksgewinde. Am distalen Ende der Kolbenstange 70 befindet sich ein knopfförmiger Abschluss der Kolbenstange 70 (Figur 2), der eine Schnappverbindung mit einem Flansch 71 ermöglicht, wodurch der Flansch 71 relativ zur Kolbenstange 70 drehbar und vorzugsweise kippbar ist, jedoch in axialer Richtung an der Kolbenstange 70 gehalten ist. Der Flansch 71 kann auf einen Stopfen 15 in der Karpule 13 einwirken, um die medizinische Substanz aus der Karpule 13 auszuschütten.

Nachfolgend wird das Einstellen und Korrigieren einer Dosis beschrieben. In der Figur 2 ist der Injektor 1 in einer Ausgangsstellung gezeigt. Zum Einstellen einer Dosis wird das Betätigungselement 60 mittels seines Knopfelements 61 relativ zum Gehäuse 10 gedreht. Da das Betätigungselement 60 mittels seiner Zähne 65 rotativ mit der Dosisanzeigehülse 30 gekoppelt ist, wird auch die Dosisanzeigehülse 30 mitgedreht. Da zudem das Betätigungselement 60 rotationsfest mit der Antriebshülse 50 verbunden ist und diese in Gewindeverbindung mit der Kolbenstange 70 steht, wird das Betätigungselement durch die Drehung in proximaler Richtung aus dem Gehäuse 10 geschraubt. Die rotativ gekoppelte Dosisanzeigehülse 30 wird ebenfalls aus dem Gehäuse 10 geschraubt, da diese mit ihrem Innengewinde in Gewindeverbindung mit der inneren Gehäusehülse 20 steht. Dabei muss das Aussengewinde 21 der Gehäusehülse die gleiche Steigung und den gleichen Gewindesinn aufweisen wie das Linksgewinde der Kolbenstange 70, da das Betätigungselement 60 und die Dosisanzeigehülse 30 nicht relativ zueinander verschoben werden sollen beim Einstellen und Korrigieren einer Dosis.

Mittels der Führungen 53, die in die axialen Nuten 64 eingreifen, sind Betätigungselement 60 und Antriebshülse 50 rotativ gekoppelt. Ein axialer Anschlag an der Antriebshülse 50 verhindert, dass das Betätigungselement 60 in proximaler Richtung über die Antriebshülse 50 abgezogen werden kann.

Figur 4a zeigt eine Schnittansicht eines mittleren Bereichs des Injektors, in welchem Bereich Antriebshülse 50 und Betätigungselement 60 miteinander verbunden sind. In der gezeigten Stellung befindet sich das Betätigungselement 60 relativ zur Antriebshülse 50 in einer proximalen Position während dem Einstellen und Korrigieren einer Dosis.

Wenn das Betätigungselement 60 relativ zur inneren Gehäusehülse 20 aus dem Gehäuse 10 geschraubt wird, werden die Nocken 63 der flexiblen Arme 62 je quer über die Längsnuten 22 geführt. Das bedeutet, bei der Relativbewegung lenken die Arme 62 kurzeitig radial nach innen. Dies ist möglich, da sich beim Einstellen und Korrigieren die Arme 62 in Längsrichtung beim abgesetzten Bereich 54 der Antriebshülse 50 befinden und somit für die radiale Auslenkung der Arme 62 Raum vorhanden ist. Beim Rotieren des Betätigungselements 60 relativ zur inneren Gehäusehülse 20 wird der flexible, elastische Arm 62 von einer Längsnut 22 zur nächsten bewegt. Dabei federt der Arm 62 jeweils in die Längsnuten 22 zurück. Dadurch wird jedes Mal wenn der Nocken 63 von einer Längsnut 22 zur nächsten bewegt wird, ein Klickgeräusch und eine Vibration erzeugt.

Wird versehentlich eine zu hohe Dosis eingestellt, kann die Dosis korrigiert werden, indem das Betätigungselement 60 wieder zurück in das Gehäuse 10 geschraubt wird. Die Nocken 63 der flexiblen Arme 62 werden dabei in Gegenrichtung über die Längsnuten 22 bewegt und erzeugen wiederum ein Klickgeräusch und eine Vibration, wenn sie von einer Längsnut 22 zur nächsten bewegt werden.

Die Stoppmutter 40 wird mittels ihrer axialen Stege axial und rotationsfest in den Längsnuten 22 der Gehäusehülse 20 geführt. Sie wird beim Einstellen einer Dosis bzw. bei einer Drehung der Antriebshülse 50 auf deren Gewinde in proximaler Richtung geschraubt. Beim Korrigieren einer Dosis bzw. Zurückdrehen des Betätigungselements 60 wird die Stoppmutter 40 entsprechend wieder in distale Richtung geschraubt. Beim Erreichen der letzten Dosis schlägt die Stoppmutter 40 proximal an die Wandung 56 der Antriebshülse 50 an und verhindert so eine weitere Drehung des Betätigungselements 60 und somit ein weiteres Aufdosieren.

Nachfolgend wird der Ausschüttvorgang beschrieben. Figur 4b zeigt eine Schnittansicht des mittleren Bereichs des Injektors, wobei sich das Betätigungselement 60 relativ zur Antriebshülse 50 in einer distalen Position befindet, wie es der Fall ist beim Ausschütten einer Dosis.

Um die eingestellte Dosis auszuschütten, drückt der Benutzer in distaler Richtung auf das Knopfelement 61. Dadurch wird das Betätigungselement 60 in distaler Richtung relativ zur Dosisanzeigehülse 30 und relativ zur Antriebshülse 50 verschoben. Hierbei werden die Zähne 65 des Betätigungselements 60 aus dem Eingriff mit den Zähnen 33 der Dosisanzeigehülse 30 herausverschoben, wodurch diese rotativ vom Betätigungselement 60 entkoppelt wird. Die distale Verschiebung relativ zur Antriebshülse 50 wird gestoppt, wenn ein distales Ende des Betätigungselements 60 an der Wandung 56 anstösst. Bei dieser Verschiebung wird der als Verriegelungselement 58 dienende zylindrische Bereich der Antriebshülse 50 unter die Arme 62 geschoben. Somit liegen die Arme 62 ganz oder nahezu ganz auf dem zylindrischen Bereich auf und sind somit radial blockiert. Die Arme 62 können nicht mehr radial nach innen ausgelenkt werden. Das bedeutet, die Nocken 63 der Arme 62 sind je in einer Längsnut 22 gehalten und können nicht mehr zur nächsten Längsnut 22 bewegt werden. Das Betätigungselement 60 und damit auch die Antriebshülse 50 sind dadurch rotationsfest mit dem Gehäuse 10 gekoppelt.

Da jedoch die Längsnuten 22 in der inneren Gehäusehülse 20 in Richtung Längsachse verlaufen, kann das Betätigungselement 60 dennoch axial relativ zum Gehäuse 10 verschoben werden. Durch die vom Benutzer aufgebrachte in distaler Richtung wirkende Kraft wird das Betätigungselement 60 und die Antriebshülse 50 weiter nach distal verschoben. Dabei überträgt die Antriebshülse 50 die Axialkraft auf das Linksgewinde der Kolbenstange 70 und zwingt die Kolbenstange 70 zur Rotation. In Folge schraubt sich diese in die nicht rotierende Antriebshülse 50 hinein. Da die Kolbenstange 70 mit ihrem Rechtsgewinde in Gewindeverbindung mit der Gehäusehülse 20 steht, schraubt sich die Kolbenstange 70 gleichzeitig in distaler Richtung. Wenn das Rechtsgewinde eine kleinere Steigung aufweist als das Linksgewinde, kann eine Untersetzung der distalen Verschiebung des Betätigungselements 60 und damit eine Kraftübersetzung erzielt werden. Der Flansch 71 verschiebt den Stopfen 15 in der Karpule 13 nach distal und das Produkt wird aus der Karpule 13 ausgeschüttet.

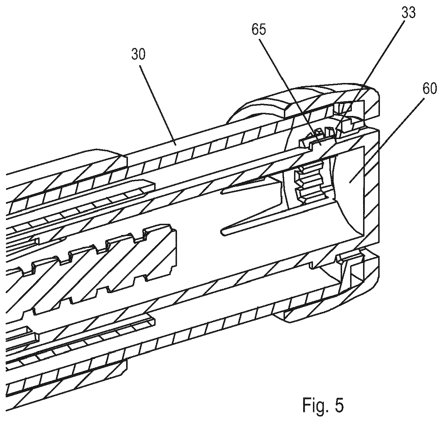

Die Figur 5 zeigt perspektivisch eine Schnittansicht des proximalen Bereichs des Injektors 1. Die Figur zeigt die Stellung nachdem der Benutzer zum Ausschütten der Dosis mit einer in distaler Richtung wirkenden Kraft auf das Knopfelement 61 gedrückt hat und somit, wie oben beschrieben, die Zähne 65 des Betätigungselements 60 aus dem Eingriff mit den Zähnen der Dosisanzeigehülse 30 herausgeschoben sind und somit Betätigungselement und Dosisanzeigehülse rotativ voneinander entkoppelt sind.

Durch die distale Verschiebung des Betätigungselements 60 relativ zum Gehäuse 10 wird die Dosisanzeigehülse 20 durch die Axialkraft zurück ins Gehäuse 10 geschraubt. Dabei bewegt sich der Klickarm 32 der Dosisanzeigehülse 30 über die Zähne 65, wodurch beim Ausschütten ein Klickgeräusch erzeugt wird. Da die Antriebshülse 50 beim Ausschütten rotationsfest zur Gehäusehülse 20 gehalten ist, wird die dazwischen angeordnete Stoppmutter 40 nicht bewegt.

In einer weiteren Ausführung des erfindungsgemässen Dosier- und Ausschüttmechanismus kann anstelle der Stoppmutter 40, welche sicherstellt, dass keine Dosis einstellbar ist, welche die Kapazität der Karpule überschreitet, ein anderer Begrenzungsmechanismus vorgesehen werden. So kann anstelle der Stoppmutter, des Aussengewindes der Kupplungshülse, der Längsnuten 22 in der Gehäusehülse 20 der erfindungsgemässe Dosier- und Ausschüttmechanismus einen Begrenzungsmechanismus zwischen Gehäusehülse 20 und Antriebshülse 50 mit exzentrischen Elementen umfassen wie er in der Patentanmeldung EP 2 918 298 A1 in den Absätzen [0059 - 0138] beschrieben und den Figuren 1 bis 25b gezeigt ist.

In einer weiteren Ausführung kann anstelle der Stoppmutter eine Kugel mit entsprechenden Führungsbahnen in der Gehäusehülse und in der Antriebshülse vorgesehen werden, wie in der Patentanmeldung WO 2010/149209 A1 auf den Seiten 25 - 27 beschrieben und in den Figuren 3 - 8 gezeigt. Alternativ kann anstelle der Kugel auch ein in Längsführungen geführtes Segment verwendet werden wie in der WO 2010/149209 A1 auf den Seiten 27 - 29 und den Figuren 9 - 14 offenbart.

### BEZUGSZEICHENLISTE

- 1: Injektor
- 10: Gehäuse
- 11: Schutzkappe
- 12: Karpulenhalter
- 13: Karpule
- 14: Öffnung
- 15: Stopfen
- 20: Gehäusehülse
- 21: Aussengewinde
- 22: Längsnuten
- 23, 24: Stege
- 30: Dosisanzeigehülse
- 31: radiale Auskragung
- 32: Klickarm
- 33: Zähne
- 40: Stoppmutter
- 50: Antriebshülse
- 51: distaler Bereich
- 52: proximaler Bereich
- 53: Führungen
- 54: abgesetzter Bereich
- 55: Spiralfeder
- 56: Wandung
- 57: Eingriffstege
- 58: Verriegelungselement
- 60: Betätigungselement
- 61: Knopfelement
- 62: Arm
- 63: Nocken
- 64: Nuten
- 65: Zähne
- 70: Kolbenstange
- 71: Flansch

## Patentansprüche

1. Ein Dosier- und Ausschüttmechanismus für eine Injektionsvorrichtung (1) zum Einstellen und Ausschütten einer Dosis eines Produkts mittels manuellem Vorschub einer Kolbenstange (70) in eine in der Injektionsvorrichtung (1) gehaltenen Karpule (13), der Dosier- und Ausschüttmechanismus umfassend
die Kolbenstange (70);
ein Gehäuse (10) mit einer Längsachse;
eine im Gehäuse (10) längsverschiebbar angeordnete Antriebshülse (50) zum Antreiben der Kolbenstange (70);
ein Betätigungselement (60) zum Einstellen der Dosis, wobei das Betätigungselement (60) rotationsfest mit der Antriebshülse (50) verbunden ist und in Richtung Längsachse relativ zur Antriebshülse (50) verschiebbar ist,
wobei zum Einstellen der Dosis das Betätigungselement (60) relativ zum Gehäuse (10) drehbar ist,
wobei das Betätigungselement (60) ein erstes, einteilig mit dem Betätigungselement (60) ausgebildetes Kopplungselement umfasst und das Gehäuse (10) ein zweites, einteilig mit dem Gehäuse (10) ausgebildetes Kopplungselement umfasst, wobei zum Ausschütten der Dosis das erste und das zweite Kopplungselement miteinander koppelbar sind, so dass im gekoppelten Zustand das Betätigungselement (60) rotationsfest mit dem Gehäuse (10) verbunden ist und in Richtung der Längsachse relativ zum Gehäuse (10) verschiebbar ist
**dadurch gekennzeichnet, dass**
die Antriebshülse (50) an einem proximalen Ende ein elastisches Element (55) umfasst und das Betätigungselement (60) mit einer durch das elastische Element (55) erzeugten Vorspannkraft in proximaler Richtung beaufschlagbar ist wobei das elastische Element (55) und die Antriebshülse (50) einteilig ausgebildet sind.

2. Dosier- und Ausschüttmechanismus nach Anspruch 1, **dadurch gekennzeichnet, dass** das erste Kopplungselement ein flexibler Arm (62) ist, welcher in radialer Richtung auslenkbar ist.

3. Dosier- und Ausschüttmechanismus nach Anspruch 2, **gekennzeichnet durch** ein Verriegelungselement (52) mit dem der flexible Arm (62) in radialer Richtung blockierbar ist.

4. Dosier- und Ausschüttmechanismus nach Anspruch 3, **dadurch gekennzeichnet, dass** durch eine Verschiebung des Betätigungselements (60) in Richtung Längsachse relativ zur Antriebshülse (50) der flexible Arm (62) mit dem Verriegelungselement (52) blockierbar ist.

5. Dosier- und Ausschüttmechanismus nach einem der Ansprüche 1 bis 4, **dadurch gekennzeichnet, dass** das zweite Kopplungselement eine Nut (22) ist, in der mindestens ein Bereich des ersten Kopplungselement aufgenommen werden kann.

6. Dosier- und Ausschüttmechanismus nach Anspruch 2 und 5, **dadurch gekennzeichnet, dass** beim Einstellen und Korrigieren einer Dosis ein freies Ende des flexiblen Arms (62) mit der Nut (22) derart zusammenwirken kann, dass ein taktiles und/oder akustisches Signal erzeugt werden kann.

7. Dosier- und Ausschüttmechanismus nach einem der Ansprüche 1 bis 6, **dadurch gekennzeichnet, dass** eine Gewindeverbindung zwischen Antriebshülse (50) und Kolbenstange (70) vorhanden ist, so dass durch eine Verschiebung der Antriebshülse (50) relativ zum Gehäuse (10) die Kolbenstange (70) in Rotation versetzt werden kann, um die Dosis auszuschütten.

8. Dosier- und Ausschüttmechanismus nach einem der Ansprüche 1 bis 7, **dadurch gekennzeichnet, dass** das Gehäuse (10) des Dosier- und Ausschüttmechanismus im Innern eine gehäusefeste innere Gehäusehülse (20) umfasst, wobei das zweite Kopplungselement auf der Innenseite der inneren Gehäusehülse (20) ausgebildet ist.

9. Dosier- und Ausschüttmechanismus nach einem der Ansprüche 1 bis 8, **dadurch gekennzeichnet, dass** der Dosier- und Ausschüttmechanismus eine im Gehäuse (10) angeordnete Dosisanzeigehülse (30) zum Anzeigen einer eingestellten Dosis umfasst, wobei die Dosisanzeigehülse (30) beim Einstellen einer Dosis mit dem Betätigungselement (60) mittels eines Kupplungsmechanismus rotationsfest gekoppelt ist.

10. Dosier- und Ausschüttmechanismus nach Anspruch 9, **dadurch gekennzeichnet, dass** beim Ausschütten die Dosisanzeigehülse (30) mittels des Kupplungsmechanismus entkoppelt ist, so dass die Dosisanzeigehülse (30) relativ zum Betätigungselement (60) rotierbar ist.

11. Dosier- und Ausschüttmechanismus nach Anspruch 9 und 10, **dadurch gekennzeichnet, dass** die Gehäusehülse (20) eine distale Rippe (23) und eine proximale Rippe (24) aufweist und dass die Dosisanzeigehülse (30) auf ihrer Innenseite ein Anschlagelement (31) aufweist, welches an die distale Rippe (23) anschlägt, um eine minimal einstellbare Dosis zu begrenzen und an die proximale Rippe (24) anschlägt, um eine maximal einstellbare Dosis zu begrenzen.

12. Dosier- und Ausschüttmechanismus nach einem der Ansprüche 9 bis 11, **dadurch gekennzeichnet, dass** die Dosisanzeigehülse (30) ein radiales Klickelement (32) umfasst, welches mit dem Betätigungselement (60) zusammenwirken kann, um ein taktiles und/oder akustisches Signal zu erzeugen.

13. Dosier- und Ausschüttmechanismus nach Anspruch 8 und 9, **dadurch gekennzeichnet, dass** die Dosisanzeigehülse (30) mit einem innenliegenden Gewinde mit der inneren Gehäusehülse (20) in Gewindeverbindung steht.

14. Eine Injektionsvorrichtung (1) mit einem Dosier- und Ausschüttmechanismus nach einem der Ansprüche 1 bis 13.

## Claims

1. A dosing and dispensing mechanism for an injection device (1) for setting and dispensing a dose of a product by means of manual advancement of a plunger (70) into a carpule (13) held in the injection device (1), the dosing and dispensing mechanism comprising
the plunger (70);
a housing (10) having a longitudinal axis;
a drive sleeve (50) longitudinally displaceably arranged in the housing (10) for driving the plunger (70);
an actuating element (60) for setting the dose, the actuating element (60) being connected to the drive sleeve (50) against rotation and being displaceable in the direction of the longitudinal axis relative to the drive sleeve (50),
the actuating element (60) being rotatable relative to the housing (10) in order to set the dose,
the actuating element (60) comprising a first coupling element formed in one piece with the actuating element (60) and the housing (10) comprising a second coupling element formed in one piece with the housing (10), the first and the second coupling element being able to be coupled to one another in order to dispense the dose, so that in the coupled state the actuating element (60) is connected to the housing (10) against rotation and is displaceable in the direction of the longitudinal axis relative to the housing (10) **characterized in that** the drive sleeve (50) comprises a resilient element (55) at a proximal end and the actuating element (60) can be acted upon in the proximal direction by a pretensioning force generated by the resilient element (55), the resilient element (55) and the drive sleeve (50) being formed in one piece.

2. Dosing and dispensing mechanism according to claim 1, **characterized in that** the first coupling element is a flexible arm (62) which can be deflected in the radial direction.

3. Dosing and dispensing mechanism according to claim 2, **characterized by** a locking element (52) with which the flexible arm (62) can be blocked in the radial direction.

4. Dosing and dispensing mechanism according to claim 3, **characterized in that** by displacement of the actuating element (60) in the direction of the longitudinal axis relative to the drive sleeve (50), the flexible arm (62) can be blocked with the locking element (52).

5. Dosing and dispensing mechanism according to any of claims 1 to 4, **characterized in that** the second coupling element is a groove (22) in which at least a portion of the first coupling element can be received.

6. Dosing and dispensing mechanism according to claims 2 and 5, **characterized in that** when a dose is set and corrected, a free end of the flexible arm (62) can interact with the groove (22) in such a way that a tactile and/or acoustic signal can be generated.

7. Dosing and dispensing mechanism according to any of claims 1 to 6, **characterized in that** a threaded connection is provided between the drive sleeve (50) and the plunger (70), so that by displacement of the drive sleeve (50) relative to the housing (10), the plunger (70) can be set in rotation in order to dispense the dose.

8. Dosing and dispensing mechanism according to any of claims 1 to 7, **characterized in that** the housing (10) of the dosing and dispensing mechanism comprises, in its interior, an inner housing sleeve (20) fixed to the housing, the second coupling element being formed on the inside of the inner housing sleeve (20).

9. Dosing and dispensing mechanism according to any of claims 1 to 8, **characterized in that** the dosing and dispensing mechanism comprises a dose indicator sleeve (30) arranged in the housing (10) for displaying a set dose, the dose indicator sleeve (30) being coupled for conjoint rotation to the actuating element (60) by means of a coupling mechanism when a dose is set.

10. Dosing and dispensing mechanism according to claim 9, **characterized in that** during dispensing, the dose indicator sleeve (30) is decoupled by means of the coupling mechanism, so that the dose indicator sleeve (30) is rotatable relative to the actuating element (60).

11. Dosing and dispensing mechanism according to claims 9 and 10, **characterized in that** the housing sleeve (20) has a distal rib (23) and a proximal rib (24) **and in that** the dose indicator sleeve (30) has on its inside a stop element (31) which abuts the distal rib (23) in order to limit a minimum settable dose and abuts the proximal rib (24) in order to limit a maximum settable dose.

12. Dosing and dispensing mechanism according to any of claims 9 to 11, **characterized in that** the dose indicator sleeve (30) comprises a radial click element (32) which can interact with the actuating element (60) to generate a tactile and/or acoustic signal.

13. Dosing and dispensing mechanism according to claims 8 and 9, **characterized in that** the dose indicator sleeve (30) is connected in a threaded manner to the inner housing sleeve (20) by means of an internal thread.

14. An injection device (1) comprising a dosing and dispensing mechanism according to any of claims 1 to 13.

## Revendications

1. Mécanisme de dosage et de distribution pour un dispositif d'injection (1) pour l'ajustement et la distribution d'une dose d'un produit au moyen d'une avance manuelle d'une tige de piston (70) dans une carpule (13) maintenue dans le dispositif d'injection (1), le mécanisme de dosage et de distribution comprenant
la tige de piston (70) ;
un boîtier (10) comportant un axe longitudinal ;
une douille d'entraînement (50) disposée de manière à pouvoir coulisser longitudinalement dans le boîtier (10) pour l'entraînement de la tige de piston (70) ;
un élément d'actionnement (60) pour l'ajustement de la dose, dans lequel l'élément d'actionnement (60) est relié de manière solidaire en rotation à la douille d'entraînement (50) et peut être coulissé en direction de l'axe longitudinal par rapport à la douille d'entraînement (50),
dans lequel, pour l'ajustement de la dose, l'élément d'actionnement (60) peut tourner par rapport au boîtier (10),
dans lequel l'élément d'actionnement (60) comprend un premier élément d'accouplement réalisé d'une seule pièce avec l'élément d'actionnement (60) et le boîtier (10) comprend un second élément d'accouplement réalisé d'une seule pièce avec le boîtier (10), dans lequel le premier et le second élément d'accouplement peuvent être accouplés l'un à l'autre pour la distribution de la dose, de sorte qu'à l'état accouplé, l'élément d'actionnement (60) est relié de manière solidaire en rotation au boîtier (10) et peut être coulissé par rapport au boîtier (10) en direction de l'axe longitudinal, **caractérisé en ce que** la douille d'entraînement (50) comprend un élément élastique (55) au niveau d'une extrémité proximale et l'élément d'actionnement (60) peut être sollicité dans la direction proximale par une force de précontrainte générée par l'élément élastique (55), dans lequel l'élément élastique (55) et la douille d'entraînement (50) sont réalisés d'une seule pièce.

2. Mécanisme de dosage et de distribution selon la revendication 1, **caractérisé en ce que** le premier élément d'accouplement est un bras flexible (62) qui peut être dévié dans une direction radiale.

3. Mécanisme de dosage et de distribution selon la revendication 2, **caractérisé par** un élément de verrouillage (52) avec lequel le bras flexible (62) peut être bloqué dans la direction radiale.

4. Mécanisme de dosage et de distribution selon la revendication 3, **caractérisé en ce que** le bras flexible (62) peut être bloqué avec l'élément de verrouillage (52) par un coulissement de l'élément d'actionnement (60) en direction de l'axe longitudinal par rapport à la douille d'entraînement (50).

5. Mécanisme de dosage et de distribution selon l'une des revendications 1 à 4, **caractérisé en ce que** le second élément d'accouplement est une rainure (22) dans laquelle peut être logée au moins une zone du premier élément d'accouplement.

6. Mécanisme de dosage et de distribution selon les revendications 2 et 5, **caractérisé en ce que,** lors de l'ajustement et de la correction d'une dose, une extrémité libre du bras flexible (62) peut coopérer avec la rainure (22) de telle sorte qu'un signal tactile et/ou acoustique peut être généré.

7. Mécanisme de dosage et de distribution selon l'une des revendications 1 à 6, **caractérisé en ce qu'**une liaison filetée est présente entre la douille d'entraînement (50) et la tige de piston (70), de sorte que la tige de piston (70) peut être mise en rotation par un coulissement de la douille d'entraînement (50) par rapport au boîtier (10) afin de distribuer la dose.

8. Mécanisme de dosage et de distribution selon l'une des revendications 1 à 7, **caractérisé en ce que** le boîtier (10) du mécanisme de dosage et de distribution comprend à l'intérieur une douille de boîtier (20) intérieure et solidaire au boîtier, dans lequel le second élément d'accouplement est réalisé sur le côté intérieur de la douille de boîtier (20) intérieure.

9. Mécanisme de dosage et de distribution selon l'une des revendications 1 à 8, **caractérisé en ce que** le mécanisme de dosage et de distribution comprend une douille indicatrice de dose (30) disposée dans le boîtier (10) pour l'indication d'une dose ajustée, dans lequel la douille indicatrice de dose (30) est accouplée de manière solidaire en rotation à l'élément d'actionnement (60) au moyen d'un mécanisme d'accouplement lors de l'ajustement d'une dose.

10. Mécanisme de dosage et de distribution selon la revendication 9, **caractérisé en ce que,** lors de la distribution, la douille indicatrice de dose (30) est désaccouplée au moyen du mécanisme d'accouplement de sorte que la douille indicatrice de dose (30) peut tourner par rapport à l'élément d'actionnement (60).

11. Mécanisme de dosage et de distribution selon les revendications 9 et 10, **caractérisé en ce que** la douille de boîtier (20) présente une nervure distale (23) et une nervure proximale (24) **et en ce que** la douille indicatrice de dose (30) présente sur son côté intérieur un élément formant butée (31) qui vient en butée contre la nervure distale (23) afin de délimiter une dose minimale ajustable et qui vient en butée contre la nervure proximale (24) afin de délimiter une dose maximale ajustable.

12. Mécanisme de dosage et de distribution selon l'une des revendications 9 à 11, **caractérisé en ce que** la douille indicatrice de dose (30) comprend un élément cliquant (32) radial pouvant coopérer avec l'élément d'actionnement (60) afin de générer un signal tactile et/ou acoustique.

13. Mécanisme de dosage et de distribution selon les revendications 8 et 9, **caractérisé en ce que** la douille indicatrice de dose (30) est en liaison filetée avec la douille de boîtier (20) intérieure par un filetage intérieur.

14. Dispositif d'injection (1) comportant un mécanisme de dosage et de distribution selon l'une des revendications 1 à 13.
